# EUROPEAN PATENT SPECIFICATION

(11) **EP 0 993 452 B1**
(45) Date of publication and mention of the grant of the patent: **05.09.2007**
(21) Application number: 98937560.5
(22) Date of filing: 22.06.1998
(51) Int. Cl.: C07D 285/08, C07D 285/135, C07D 417/04, A61K 31/41, A61K 31/495, C07D 213/83, C07D 241/28, C07D 295/155

(54) **ANGIOGENESIS INHIBITING 5-SUBSTITUTED-1,2,4-THIADIAZOLYL DERIVATIVES**
ANGIOGENESE-HEMMENDE 5-SUBSTITUIERTE-1,2,4-THIADIAZOLYLDERIVATE
DERIVES DE 5-SUBSTITUES-1,2,4-THIADIAZOLYLE INHIBANT ANGIOGENESE

(30) Priority: 24.06.1997 EP 97201931; 10.07.1997 US 53003 P
(43) Date of publication of application: 19.04.2000
(73) Proprietor: JANSSEN PHARMACEUTICA N.V., 2340 Beerse (BE)
(72) Inventor: STOKBROEKX, Raymond, Antoine, B-2340 Beerse (BE); CEUSTERS, Marc, André, B-3293 Diest (BE); VAN DER AA, Marcel, Jozef, Maria, B-2300 Turnhout (BE); LUYCKX, Marcel, Gerebernus, Maria, B-2440 Geel (BE); WILLEMS, Marc, B-2350 Vosselaar (BE); TUMAN, Robert, W., Chalfont, PA 18914 (US)
(74) Representative: Daelemans, Frank F.R.
(86) International application number: PCT/EP1998/004022
(87) International publication number: WO 1998/058919

(56) References cited:
- WO-A-97/26258
- US-A- 5 104 889

## Description

This invention concerns 5-substituted-1,2,4-thiadiazolyl derivatives acting as angiogenesis inhibitors, and their preparation; it further relates to compositions comprising them, as well as their use as a medicine.

Angiogenesis, i.e. the formation of new vessels by endothelial cells, plays an important role in a variety of physiologic and pathophysiologic processes. The development of a vascular supply is essential for the growth, maturation and maintenance of normal tissues. It is also required for wound healing. Angiogenesis is critical for solid tumor growth and metastasis and is involved in a variety of other pathological conditions such as neovascular glaucoma, diabetic retinopathy, psoriasis and rheumatoid arthritis. These pathological states are characterized by augmented angiogenesis during which normally quiescent endothelial cells become activated, degrade extracellular matrix barriers, proliferate, and migrate to form new vessels. To control these angiogenesis dependent disorders, compounds with angiogenesis inhibitory properties would be very useful.

Several compounds inhibiting angiogenesis, also called angiostatics, angio-inhibitors or angiogenic antagonists, are disclosed in the art. For instance hydrocortisone is a well known angiogenesis inhibitor (Folkman et al., Science 230:1375, 1985' "A new class of steroids inhibits angiogenesis in the presence of heparin or a heparin fragment"; Folkman et al., Science 221:719, 1983, "Angiogenesis inhibition and tumor regression caused by heparin or a heparin fragment in the presence of cortisone").

EP-0,398,427, publised on November 22 1990, discloses antirhinoviral pyridazinames, and in EP-0,435,381, published on July 3 1991, pyridazinamines are described having antipicornaviral activity. EP-0,429,344, published on May 29 1991, discloses aminopyridazine derivatives as cholinergic agonists.

The compounds of the present invention differ from the prior art compounds by the fact that they are invariably substituted with a thiadiazolyl moiety and particularly by the fact that unexpectedly these compounds have angiogenesis inhibiting properties.

This invention concerns compounds of formula 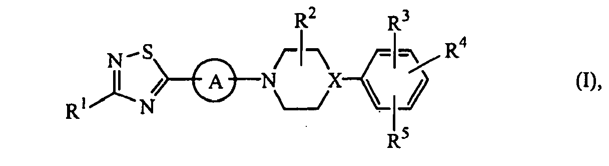 the *N*-oxide forms, the pharmaceutically acceptable acid addition salts and stereochemically isomeric forms thereof, wherein
- X: is CH or N;
- R¹: is hydrogen, C₁₋₆alkyl, C₁₋₆alkyloxy, C₁₋₆alkylthio, amino, mono- or di(C₁₋₆alkyl)amino, Ar¹, Ar¹NH-, C₃₋₆cycloalkyl, hydroxymethyl or benzyloxymethyl;
- R²: is hydrogen;
- R³, R⁴ and R⁵: are each independently selected from hydrogen, halo, C₁₋₆alkyl, C₁₋₆alkyloxy, trifluoromethyl, nitro, amino, cyano, azido, C₁₋₆alkyloxyC₁₋₆alkyl, C₁₋₆alkylthio, C₁₋₆alkyloxycarbonyl or Het¹; 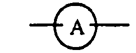 is Ar², Ar²CH₂- or Het²;
- Ar¹: is phenyl; phenyl substituted with 1, 2 or 3 substituents each independently selected from halo, C₁₋₆alkyl, C₁₋₆alkyloxy, trihalomethyl, amino or nitro;
- Ar²: is phenyl;
- Het¹: is a monocyclic heterocycle selected from oxazolyl, isoxazolyl, oxadiazolyl, thiazolyl, isothiazolyl, thiadiazolyl or oxazolinyl; and each monocyclic heterocycle may optionally be substituted on a carbon atom with C₁₋₄alkyl; and
- Het²: is a monocyclic heterocycle selected from thiadiazolyl, pyridinyl, pyrimidinyl or pyrazinyl.

As used in the foregoing definitions and hereinafter, halo is generic to fluoro, chloro, bromo and iodo; C₁₋₄alkyl defines straight and branched chain saturated hydrocarbon radicals having from 1 to 4 carbon atoms such as, for example, methyl, ethyl, propyl, butyl, 1-methylethyl, 2-methylpropyl and the like; C₁₋₆alkyl is meant to include C₁₋₄alkyl and the higher homologues thereof having 5 to 6 carbon atoms such as, for example, pentyl, 2-methylbutyl, hexyl, 2-methylpentyl and the like. Examples of the 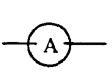 moiety are

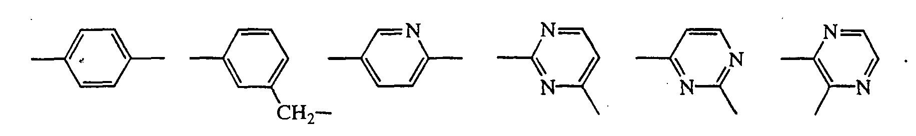

Wherever 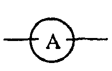 is a radical of formula Ar²CH₂, the CH₂ moiety of said radical preferably is connected to the nitrogen atom of the piperidinyl moiety when X is CH, or piperazinyl moiety when X is nitrogen.

The pharmaceutically acceptable acid addition salts as mentioned hereinabove are meant to comprise the therapeutically active non-toxic acid addition salt forms which the compounds of formula (I) are able to form. The compounds of formula (I) which have basic properties can be converted in their pharmaceutically acceptable acid addition salts by treating said base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic (i.e. butanedioic acid), maleic, fumaric, malic, tartaric, citric, methanesulfonic, ethanesulfonic, benzenesulfonic, *p*-toluenesulfonic, cyclamic, salicylic, *p*-amino-salicylic, pamoic and the like acids.

The term acid addition salts also comprises the hydrates and the solvent addition forms which the compounds of formula (I) are able to form. Examples of such forms are e.g. hydrates, alcoholates and the like.

The term stereochemically isomeric forms of compounds of formula (I), as used hereinbefore, defines all possible compounds made up of the same atoms bonded by the same sequence of bonds but having different three-dimensional structures which are not interchangeable, which the compounds of formula (I) may possess. Unless otherwise mentioned or indicated, the chemical designation of a compound encompasses the mixture of all possible stereochemically isomeric forms which said compound may possess. Said mixture may contain all diastereomers and/or enantiomers of the basic molecular structure of said compound. All stereochemically isomeric forms of the compounds of formula (I) both in pure form or in admixture with each other are intended to be embraced within the scope of the present invention.

Some of the compounds of formula (I) may also exist in their tautomeric forms. Such forms although not explicitly indicated in the above formula are intended to be included within the scope of the present invention.

The *N*-oxide forms of the compounds of formula (I) are meant to comprise those compounds of formula (I) wherein one or several nitrogen atoms are oxidized to the so-called *N*-oxide.

Whenever used hereinafter, the term "compounds of formula (I)" is meant to include also the pharmaceutically acceptable acid addition salts and all stereoisomeric forms.

A group of interesting compounds consists of those compounds of formula (I) wherein one or more of the following restrictions apply :
a) X is N;
b) R¹ is hydrogen, C₁₋₆alkyl, amino or di(C₁₋₆alkyl)amino;
c) R² is hydrogen;
d) R³, R⁴ and R⁵ are each independently selected from hydrogen, halo, C₁₋₆alkyl, C₁₋₆alkyloxy, trifluoromethyl, nitro or C₁₋₆alkyloxycarbonyl.

A particular group of compounds are those compounds of formula (I) wherein X is N; R¹ is hydrogen, C₁₋₄alkyl or di(C₁₋₄alkyl)amino; R² is hydrogen; R³, R⁴ and R⁵ are each independently selected from hydrogen, halo, C₁₋₄alkyl, C₁₋₄alkyloxy or trifluoromethyl; and the bivalent radical 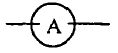 is Ar², Ar²CH₂- or Het² wherein Ar² is phenyl, or phenyl substituted with 1 or 2 substituents each independently selected from halo, C₁₋₆alkyl, C₁₋₆alkyloxy, trihalomethyl, amino or nitro; and Het² is thiadiazolyl, pyridinyl, pyrimidinyl or pyrazinyl.

A preferred group of compounds are those compounds of formula (I) wherein X is N, R¹ is methyl, R² is hydrogen, R³ and R⁴ are hydrogen and R⁵ is trifluoromethyl.

A more preferred group of compounds are those preferred compounds wherein R⁵ is trifluoromethyl situated on the 3-position.

Most preferred are :
1-[4-(3-methyl-1,2,4-thiadiazol-5-yl)phenyl]-4-[3-(trifluoromethyl)phenyl]piperazine, and
1-[5-(3-methyl-1,2,4-thiadiazol-5-yl)-2-pyridinyl]-4-[3-(trifluoromethyl)-phenyl]-piperazine, and the pharmaceutically acceptable acid addition salts, the stereoisomeric forms, or the *N*-oxides thereof.

The compounds of the present invention can generally be prepared by reacting an intermediate of formula (II) with an intermediate of formula (III).

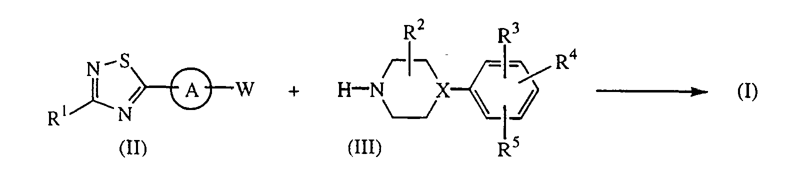

In the foregoing and following reaction schemes W represents an appropriate reactive leaving group such as, for example, halo, e.g. fluoro, chloro, bromo, iodo, or in some instances W may also be a sulfonyloxy group, e.g. methanesulfonyloxy, benzene-sulfonyloxy, trifluoromethanesulfonyloxy and the like reactive leaving groups. Said reaction is performed following art-known procedures such as for instance stirring both reactants together in a reaction-inert solvent, e.g. *N,N-*dimethylformamide, acetonitrile, methyl isobutylketone and the like, preferably in the presence of a base, e.g. sodium hydrogen carbonate, sodiumcarbonate or triethylamine. The reaction may conveniently be carried out at a temperature ranging between room temperature and the reflux temperature of the reaction mixture.

The compounds of formula (I) wherein R¹ is CH₃, said compounds being represented by formula (I-a) can also be prepared by treating an intermediate of formula (IV) with hydroxylamino-O-sulfonic acid in a reaction-inert solvent such as, e.g. methanol or ethanol, in the presence of a base such as, e.g. pyridine.

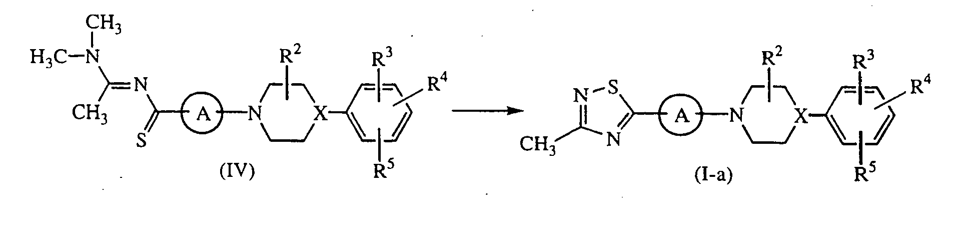

The compounds of formula (I) may further be prepared by converting compounds of formula (I) into each other according to art-known group transformation reactions.

The compounds of formula (I) may also be converted to the corresponding *N*-oxide forms following art-known procedures for converting a trivalent nitrogen into its *N*-oxide form. Said *N*-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *tert*-butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

The starting materials and some of the intermediates are known compounds and are commercially available or may be prepared according to conventional reaction procedures generally known in the art. For instance, some intermediates of formula (II), such as 5-(4-fluorophenyl)-3-methyl-1,2,4-thiadiazole, have been described by Yang-i Lin et al. in J. Org. Chem., 45(19), p. 3750 - 3753 (1980), and some intermediates of formula (III), such as 1-[3-(trifluoromethyl)phenyl]-piperazine, are commercially available.

Intermediates of formula (II) may be prepared by reacting compounds of formula (V), wherein W is an appropriate leaving group as defined above, with an intermediate of formula (VI), optionally added as its acid addition salt.

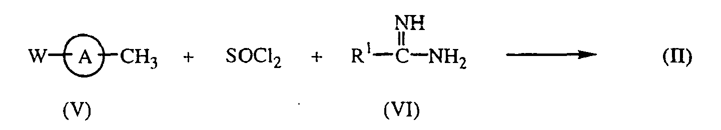

Intermediates of formula (IV) can be prepared as outlined in scheme I.

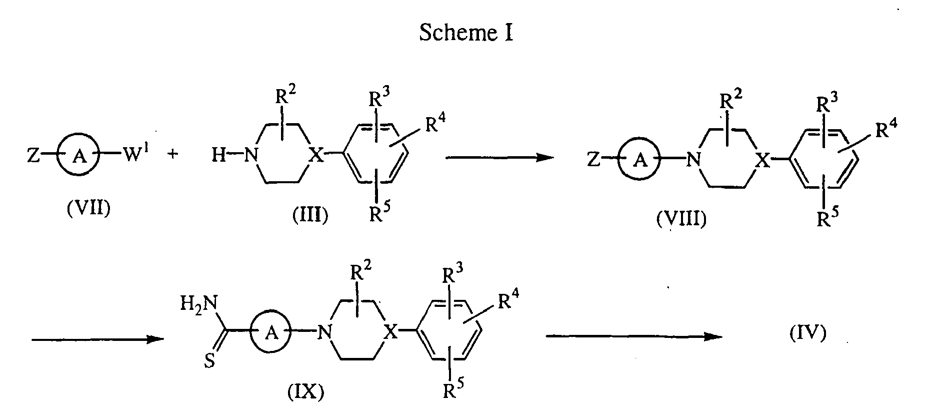

In scheme I, an intermediate of formula (VII), wherein W¹ is an appropriate leaving group such as halo or sulfonyloxy and Z is cyano or aminocarbonyl, is reacted with an intermediate of formula (III) under art-known reaction procedures as described hereinabove for the synthesis of compounds of formula (I). The resulting intermediates of formula (VIII) are treated with Lawesson's reagent in a suitable solvent such as, for example, toluene or pyridine, or are treated with H₂S in a suitable solvent such as, e.g. *N*,*N*-dimethylformamide, optionally in the presence of triethylamine. Subsequently, intermediates of formula (IX) are treated with *N*,*N*-dimethylacetamide dimethyl acetal in a reaction-inert solvent such as, e.g. toluene or dichloromethane, thereby yielding intermediates of formula (IV).

Compounds of formula (I) and some of the intermediates may have one or more stereogenic centers in their structure, present in a R or a S configuration. For instance, R¹, R², R³, R⁴ or R⁵ can be a C₁₋₆alkyl having a stereogenic center.

The compounds of formula (I) as prepared in the hereinabove described processes may be synthesized in the form of racemic mixtures of enantiomers which can be separated from one another following art-known resolution procedures. The racemic compounds of formula (I) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of formula (I) involves liquid chromatography using a chiral stationary phase. Suitable chiral stationary phases are, for example, polysaccharides, in particular cellulose or amylose derivatives. Commercially available polysaccharide based chiral stationary phases are ChiralCel CA, OA, OB, OC, OD, OF, OG, OJ and OK, and Chiralpak AD, AS, OP(+) and OT(+). Appropriate eluents or mobile phases for use in combination with said polysaccharide chiral stationary phases are hexane and the like, modified with an alcohol such as ethanol, isopropanol and the like. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds of formula (I) have valuable pharmacological properties in that they inhibit angiogenesis, both *in vivo* and *in vitro.*

In view of their pharmacological activity, the compounds of formula (I), their pharmaceutically acceptable acid addition salts, stereochemically isomeric forms, or *N*-oxide forms thereof, are inhibitors of angiogenesis. Therefore, angiogenesis inhibitors are useful to control or treat angiogenesis dependent disorders such as, e.g. ocular neovascular diseases, neovascular glaucoma, diabetic retinopathy, retrolental fibroplasia, hemangiomas, angiofibromas, psoriasis, osteoarthritis and rheumatoid arthritis. Also, angiogenesis inhibitors are useful to control solid tumor growth, such as, e.g. breast, prostate, melanoma, renal, colon, cervical cancer and the like; and metastasis.

Hence, the present invention discloses the compounds of formula (I) for use as a medicine as well as the use of these compounds of formula (I) for the manufacture of a medicament for treating angiogenesis dependent disorders.

In view of the usefulness of the subject compounds in the treatment or prevention of angiogenesis dependent disorders, the present invention provides a method of treating warm-blooded animals suffering from such disorders, said method comprising the systemic administration of a therapeutic effective amount of a compound of formula (I), a *N*-oxide or a pharmaceutically acceptable acid addition salt thereof.

In view of their useful pharmacological properties, the subject compounds may be formulated into various pharmaceutical forms for administration purposes. To prepare the pharmaceutical compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions.
It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

For oral administration, the pharmaceutical compositions may take the form of solid dose forms, for example, tablets (both swallowable-only and chewable forms), capsules or gelcaps, prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium phosphate); lubricants e.g. magnesium stearate, talc or silica); disintegrants (e.g. potato starch or sodium starch glycollate); or wetting agents (e.g. sodium lauryl sulphate). The tablets may be coated by methods well known in the art.

Liquid preparations for oral administration may take the form of, for example, solutions, syrups or suspensions, or they may be presented as a dry product for constitution with water or other suitable vehicle before use. Such liquid preparations may be prepared by conventional means, optionally with pharmaceutically acceptable additives such as suspending agents (e.g. sorbitol syrup, methylcellulose, hydroxypropyl methylcellulose or hydrogenated edible fats); emulsifying agents (e.g. lecithin or acacia); non-aqueous vehicles (e.g. almond oil, oily esters or ethyl alcohol); and preservatives (e.g. methyl or propyl p-hydroxybenzoates or sorbic acid).

Pharmaceutically acceptable sweeteners comprise preferably at least one intense sweetener such as saccharin, sodium or calcium saccharin, aspartame, acesulfame potassium, sodium cyclamate, alitame, a dihydrochalcone sweetener, monellin, stevioside or sucralose (4,1',6'-trichloro-4,1',6'-trideoxy*galacto*sucrose), preferably saccharin, sodium or calcium saccharin, and optionally a bulk sweetener such as sorbitol, mannitol, fructose, sucrose, maltose, isomalt, glucose, hydrogenated glucose syrup, xylitol, caramel or honey.

Intense sweeteners are conveniently employed in low concentrations. For example, in the case of sodium saccharin, the concentration may range from 0.04% to 0.1% (w/v) based on the total volume of the final formulation, and preferably is about 0.06% in the low-dosage formulations and about 0.08% in the high-dosage ones. The bulk sweetener can effectively be used in larger quantities ranging from about 10% to about 35%, preferably from about 10% to 15% (w/v).

The pharmaceutically acceptable flavours which can mask the bitter tasting ingredients in the low-dosage formulations are preferably fruit flavours such as cherry, raspberry, black currant or strawberry flavour. A combination of two flavours may yield very good results. In the high-dosage formulations stronger flavours may be required such as Caramel Chocolate flavour, Mint Cool flavour, Fantasy flavour and the like pharmaceutically acceptable strong flavours. Each flavour may be present in the final composition in a concentration ranging from 0.05% to 1% (w/v). Combinations of said strong flavours are advantageously used. Preferably a flavour is used that does not undergo any change or loss of taste and colour under the acidic conditions of the formulation.

The compounds of the invention may also be formulated as depot preparations. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example as a sparingly soluble salt.

The compounds of the invention may be formulated for parenteral administration by injection, conveniently intravenous, intramuscular or subcutaneous injection, for example by bolus injection or continuous intravenous infusion. Formulations for injection may be presented in unit dosage form e.g. in ampoules or in multidose containers, with an added preservative. The compositions may take such forms as suspensions, solutions or emulsions in oily or aqueous vehicles, and may contain formulatory agents such as isotonizing, suspending, stabilising and/or dispersing agents. Alternatively, the active ingredient may be in powder form for constitution with a suitable vehicle, e.g. sterile pyrogen-free water before use.

The compounds of the invention may also be formulated in rectal compositions such as suppositories or retention enemas, e.g. containing conventional suppository bases such as cocoa butter or other glycerides.

For intranasal administration the compounds of the invention may be used, for example, as a liquid spray, as a powder or in the form of drops.

Those skilled in the art could easily determine the effective amount from the test results presented hereinafter. In general it is contemplated that an effective amount would be from 0.001 mg/kg to 10 mg/kg body weight, and in particular from 0.01 mg/kg to 1 mg/kg body weight. It may be appropriate to administer the required dose as two, three, four or more sub-doses at appropriate intervals throughout the day. Said sub-doses may be formulated as unit dosage forms, for example, containing 0.01 to 500 mg, and in particular 0.1 mg to 200 mg of active ingredient per unit dosage form.

The following examples are provided for purposes of illustration

### Experimental part

Hereinafter "DMF" means *N,N*-dimethylformamide, "DCM" means dichloromethane, "DIPE" means diisopropylether and "THF" means tetrahydrofuran.

### A. Preparation of the intermediates

### Example A.1

a) A mixture of 2-chloro-4-methylpyrimidinyl (0.07 mol) in thionyl chloride (100 ml) was stirred and refluxed for 16 hours. The solvent was evaporated yielding 2-chloro-4-[dichloro(chlorothio)methyl]pyrimidine (intermediate 1).
b) 1-Imino-ethanamine hydrochloride (1:1) (0.08 mol) was added at 0°C to a stirring mixture of intermediate 1 (0.07 mol) in DCM (300 ml). Sodium hydroxide (50%, 20 ml) was added dropwise at 0°C. The mixture was stirred at 5°C for 1 hour. Water (300 ml) and DCM (300 ml) were added. The mixture was separated into its layers. The aqueous layer was washed twice with DCM. The combined organic layer was dried, filtered and the solvent was evaporated. The residue was purified over silica gel on a glass filter (eluent : DCM). Two pure fractions were collected and their solvents were evaporated, yielding 3.5g of 2-chloro-4-(3-methyl-1,2,4-thiadiazol-5-yl)pyrimidine (intermediate 2).

### Example A.2

a) A mixture of 6-chloro-3-pyridinecarboxamide (0.11 mol), 1-[3-(trifluoromethyl)-phenyl]-piperazine (0.11 mol) and sodium carbonate (0.22 mol) in DMF (300 ml) was stirred at 120°C overnight. The mixture was poured out into ice water (600ml) and stirred for 1 hour. The precipitate was filtered off and dried Part of this fraction (4 g) was taken up in DCM and an aqueous NaHCO₃ solution. The mixture was separated into its layers. The aqueous layer was extracted three times with DCM. The combined organic layer was dried, filtered and the solvent was evaporated till a small volume. The precipitate was filtered off and dried, yielding 3.2 g of 6-[4-[3-(trifluoromethyl)phenyl]-1-piperazinyl]-3-pyridinecarboxamide (intermediate 3).
b) A mixture of intermediate 3 (0.013 mol) and Lawesson's reagens (0.007 mol) in toluene (130 ml) was stirred and refluxed for 2 hours. The mixture was cooled. Water (100 ml) was added. The mixture was stirred for 1 hour and separated into its layers.

The aqueous layer was extracted three times with toluene and once with DCM. The combined organic layer was dried, filtered and the solvent was evaporated, yielding: 7.3g of 6-[4-[3-(trifluoromethyl)phenyl]-1-piperazinyl]-3-pyridinecarbothioamide (intermediate 4).

### Example A.3

A mixture of intermediate 4 (0.013 mol) and 1,1-dimethoxy-*N*,*N*-dimethyl-ethanamine (0.021 mol) was allowed to stand overnight and then used without further purification, yielding *N*-[1-(dimethylamino)ethylidene]-6-[4-[3-(trifluoromethyl)phenyl]-1-piperazinyl]-3-pyridinecarbothioamide (intermediate 5).
Table I.1 lists the intemediates that were prepared according to example A.3.

**Table I.1 :**

| | | | |
|---|---|---|---|
| 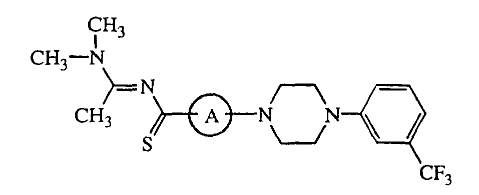 | | | |

| Intm. No. | Ex. No. | 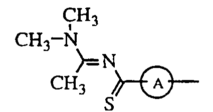 | Physical data |
|---|---|---|---|
| 5 | A.3 | 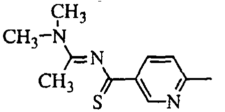 | - |
| 6 | A.3 | 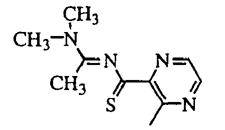 | mp. 156°C |
| 7 | A.3 | 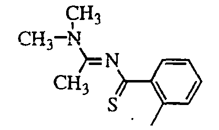 | - |
| 8 | A.3 | 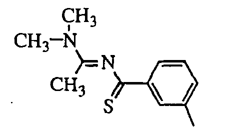 | - |
| 9 | A.3 | 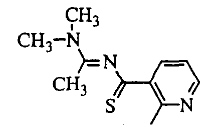 | - |
| 10 | A.3 | 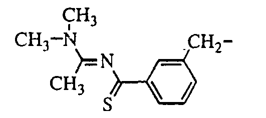 | - |

### B. Preparation of the final compounds

### Example B.1

A mixture of 5-(4-fluorophenyl)-3-methyl-1,2,4-thiadiazole (0.012 mol), 1-[3-(trifluoromethyl)phenyl]-piperazine (0.414 mol) and sodium carbonate (0.024 mol) in DMF (10 ml) was stirred at 140°C for 24 hours, then at 150°C for 24 hours, cooled, poured out into ice water (200 ml) and stirred. The precipitate was filtered off, taken up in DCM, dried, filtered and the solvent was evaporated. The residue was crystallized from DIPE. The precipitate was filtered off and dried, yielding 2.5g (52%) of 1-[4-(3-methyl-1,2,4-thiadiazol-5-yl)phenyl]-4-[3-(trifluoromethyl)phenyl]piperazine (compound 2).

### Example B.2

A mixture of hydroxylamine-*O*-sulfonic acid (0.011 mol) in methanol (15 ml) was added at once to a mixture of intermediate 5 (0.01 mol) and pyridine (0.02 mol) in ethanol (40 ml). The mixture was stirred at room temperature for 90 minutes. The solvent was evaporated. The residue was dissolved in DCM, washed with water and an aqueous NaOH 0.1N solution, dried, filtered and the solvent was evaporated. The residue was taken up in methanol, filtered off and dried. The residue was taken up in acetonitrile (100 ml). The mixture was stirred and boiled until complete dissolution and then allowed to crystallize out. The precipitate was filtered off and dried, yielding 1.4 g (35%) of 1-[5-(3-methyl-1,2,4-thiadiazol-5-yl)-2-pyridinyl]-4-[3-(trifluoromethyl)-phenyl]piperazine (compound 8).

Table F.1 lists the compounds that were prepared according to one of the above examples and table F.2 lists both the experimental (column heading "exp.") and theoretical (column heading "theor.") elemental analysis values for carbon, hydrogen and nitrogen of the compounds as prepared in the experimental part hereinabove.

**Table F.1 :**

| | | | |
|---|---|---|---|
| 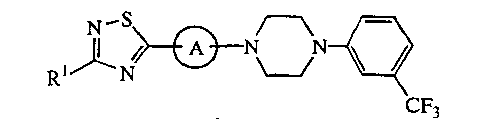 | | | |

| Co. No. | Ex. No. | 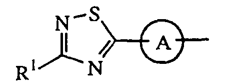 | Physical data |
|---|---|---|---|
| 1 | B.1 | 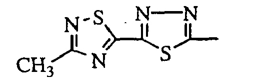 | mp. 184.8°C |
| 2 | B.1 | 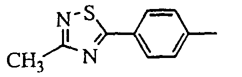 | - |
| 3 | B.2 | 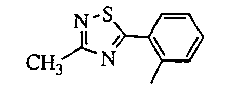 | - |
| 4 | B.2 | 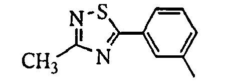 | .HCl (1:1) |
| 5 | B.2 | 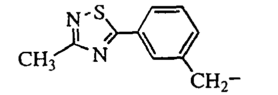 | .HCl (1:1) |
| 6 | B.2 | 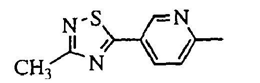 | - |
| 7 | B.2 | 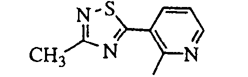 | - |
| 8 | B.1 | 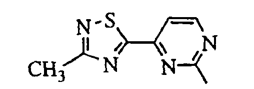 | - |
| 9 | B.1 | 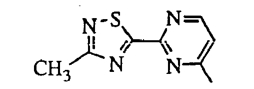 | mp. 140°C |
| 10 | B.2 | 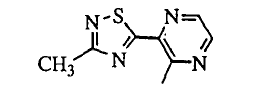 | mp. 140°C |

**Table F.2 :**

| Comp. No. | Carbon | | Hydrogen | | Nitrogen | |
|---|---|---|---|---|---|---|
| | Exp. | Theor. | Exp. | Theor. | Exp. | Theor. |
| 2 | 59.39 | 59.31 | 4.73 | 4.68 | 13.85 | 13.88 |
| 3 | 59.39 | 59.32 | 4.73 | 4.67 | 13.85 | 13.92 |
| 5 | 55.44 | 55.29 | 4.87 | 4.96 | 12.32 | 12.39 |
| 6 | 56.29 | 55.30 | 4.47 | 4.33 | 17.27 | 17.06 |
| 7 | 56.29 | 56.24 | 4.47 | 4.45 | 17.27 | 17.43 |
| 8 | 53.19 | 52.21 | 4.22 | 4.07 | 20.68 | 20.33 |

### C. Pharmacological examples

### Example C. 1

Angiogenesis inhibitory activity was measured *in vitro* using the rat aortic ring model of angiogenesis as described by Nicosia, R.F. and Ottinetti in "Laboratory Investigation", vol. 63, p. 115, 1990. The ability of compounds to inhibit microvessel formation was compared to vehicle-treated control rings. Quantitation (microvessel area) following eight days in culture was performed using an image analysis system, consisting of a light microscope, a CCD camera and an automated, custom-designed image analysis program as described by Nissanov, J., Tuman, R.W., Gruver, L.M., and Fortunato, J.M. in "Laboratory Investigation", vol 73 (#5), p. 734, 1995. Compounds were tested at several concentrations for determination of inhibitory potency (IC₅₀'s). Compounds 1, 2 and 6 have an IC₅₀ value lower than 10 nM.

## Claims

1. A compound of formula (I), 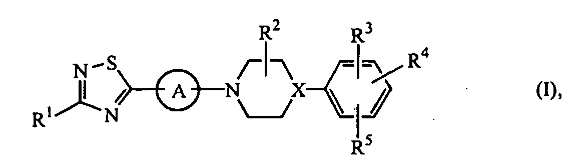 the *N*-oxide forms, the pharmaceutically acceptable acid addition salts and stereochemically isomeric forms thereof, wherein
X is CH or N;
R¹ is hydrogen, C₁₋₆alkyl, C₁₋₆alkyloxy, C₁₋₆alkylthio, amino, mono- or di(C₁₋₆alkyl)amino, Ar¹, Ar¹NH-, C₃₋₆cycloalkyl, hydroxymethyl or benzyloxymethyl;
R² is hydrogen;
R³, R⁴ and R⁵ are each independently selected from hydrogen, halo, C₁₋₆alkyl, C₁₋₆alkyloxy, trifluoromethyl, nitro, amino, cyano, azido, C₁₋₆alkyloxyC₁₋₆alkyl, C₁₋₆alkylthio, C₁₋₆alkyloxycarbonyl or Het¹ ; 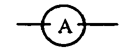 is Ar², Ar²CH₂- or Het²;
Ar¹ is phenyl; phenyl substituted with 1, 2 or 3 substituents each independently selected from halo, C₁₋₆alkyl, C₁₋₆alkyloxy, trihalomethyl, amino or nitro;
Ar² is phenyl;
Het¹ is a monocyclic heterocycle selected from oxazolyl, isoxazolyl, oxadiazolyl, thiazolyl, isothiazolyl, thiadiazolyl or oxazolinyl; and each monocyclic heterocycle may optionally be substituted on a carbon atom with C₁₋₄alkyl; and
Het² is a monocyclic heterocycle selected from thiadiazolyl, pyridinyl, pyrimidinyl or pyrazinyl.

2. A compound according to claim 1 wherein X is N; R¹ is hydrogen, C₁₋₆alkyl, amino or di(C₁₋₆alkyl)amino; R² is hydrogen; R³, R⁴ and R⁵ are each independently selected from hydrogen, halo, C₁₋₆alkyl, C₁₋₆alkyloxy, trifluoromethyl, nitro or C₁₋₆alkyloxycarbonyl.

3. A compound according to any of claims 1 or 2 wherein X is N; R¹ is hydrogen, C₁₋₄alkyl or di(C₁₋₄alkyl)amino; R² is hydrogen; R³, R⁴ and R⁵ are each independently selected from hydrogen, halo, C₁₋₄alkyl, C₁₋₄alkyloxy or trifluoromethyl; and the bivalent radical 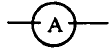 is Ar², Ar²CH₂- or Het² wherein Ar² is phenyl and Het² is thiadiazolyl, pyridinyl, pyrimidinyl or pyrazinyl.

4. A compound according to any of claims 1 to 3 wherein X is N, R¹ is methyl, R² is hydrogen, R³ and R⁴ are hydrogen and R⁵ is trifluoromethyl

5. A compound according to claim 1 wherein the compound is 1-[4-(3-methyl-1,2,4-thiadiazol-5-yl)phenyl]-4-[3-(trifluoromethyl)phenyl]-piperazine; or
1-[5-(3-methyl-1,2,4-thiadiazol-5-yl)-2-pyridinyl]-4-[3-(trifluoromethyl)phenyl]-piperazine; a stereoisomeric form or a pharmaceutically acceptable acid addition salt thereof.

6. A composition comprising a pharmaceutically acceptable carrier, and as active ingredient a therapeutically effective amount of a compound as claimed in any one of claims 1 to 5.

7. A process of preparing a pharmaceutical composition as claimed in claim 6 wherein the pharmaceutically acceptable carriers and a compound as claimed in claim 1 to 5 are intimately mixed.

8. A compound as claimed in any one of claims 1 to 5 for use as a medicine.

9. Use of a compound as claimed in any one of claims 1 to 5 for the manufacture of a medicament for the treatment of angiogenesis dependent disorders.

10. A process of preparing a compound as claimed in claim 1, wherein
a) an intermediate of formula (II) is reacted with an intermediate of formula (III) in a reaction-inert solvent and, optionally in the presence of a suitable base; 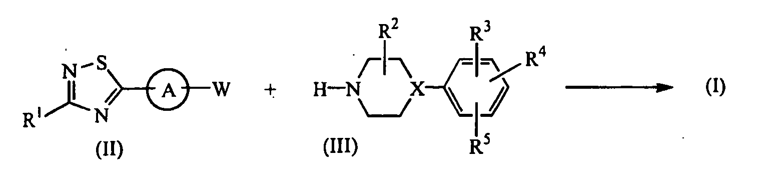
b) an intermediate of formula (IV) is treated with hydroxylamino-O-sulfonic acid in a reaction-inert solvent, in the presence of a suitable base, thereby yielding compounds of formula (I-a), defined as compounds of formula (I) wherein R¹ is methyl; 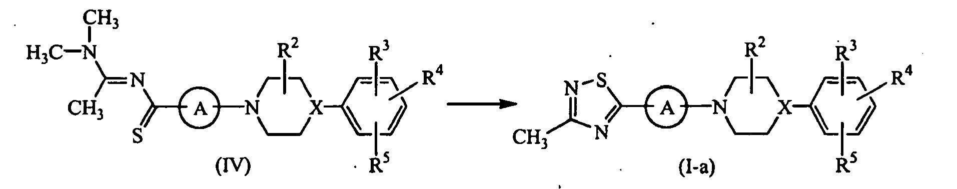
wherein in the above reaction schemes the radicals X, R¹, R², R³, R⁴, R⁵ and 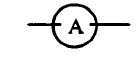 are as defined in claim 1, and W is an appropriate leaving group.

11. A compound of formula (IV), 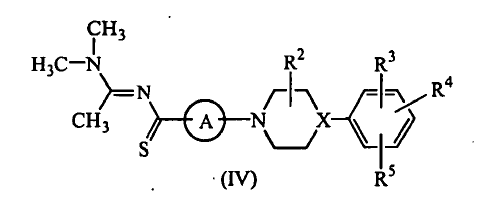 an acid addition salt, a *N-*oxide form or a stereochemically isomeric form thereof, wherein X, R², R³, R⁴, R⁵ and the bivalent radical 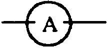 are as defined in claim 1.

12. A process of preparing a compound of formula (IV) as claimed in claim 10, wherein
a) an intermediate of formula (IX) is treated with *N,N-*dimethylacetamide dimethyl acetal in a reaction-inert solvent, thereby yielding a compound of formula (IV).

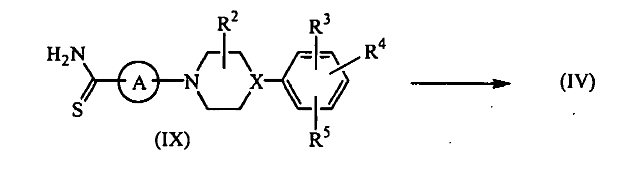

## Patentansprüche

1. Verbindungen der Formel (I) 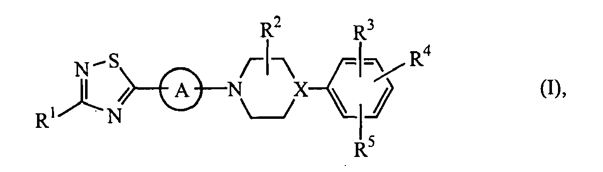 deren *N*-Oxidformen, deren pharmazeutisch unbedenkliche Säureadditionssalze und deren stereochemisch isomere Formen, wobei
X für CH oder N steht;
R¹ für Wasserstoff, C₁₋₆-Alkyl, C₁₋₆-Alkyloxy, C₁₋₆-Alkylthio, Amino, Mono- oder Di(C₁₋₆-alkyl)amino, Ar¹, Ar¹NH-, C₃₋₆-Cycloalkyl, Hydroxymethyl oder Benzyloxymethyl steht;
R² für Wasserstoff steht;
R³, R⁴ und R⁵ jeweils unabhängig voneinander aus Wasserstoff, Halogen, C₁₋₆-Alkyl, C₁₋₆-Alkyloxy, Trifluormethyl, Nitro, Amino, Cyano, Azido, C₁-6-Alkyloxy-C₁₋₄-alkyl, C₁₋₆-Alkythio, C₁₋₆-Alkyloxycarbonyl oder Het¹ ausgewählt sind; 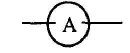 für Ar², Ar²CH₂- oder Het² steht;
Ar¹ für Phenyl oder durch 1, 2 oder 3 Substituenten jeweils unabhängig voneinander ausgewählt aus Halogen, C₁₋₆-Alkyl , C₁₋₆-Alkyloxy, Trihalogenmethyl, Amino und Nitro substituiertes Phenyl steht;
Ar² für Phenyl steht;
Het¹ für einen monocyclischen Heterocyclus ausgewählt aus Oxazolyl, Isoxazolyl, Oxadiazolyl, Thiazolyl, Isothiazolyl, Thiadiazolyl und Oxazolinyl steht; und der monocyclische Heterocyclus jeweils gegebenenfalls an einem Kohlenstoffatom durch C₁₋₄-Alkyl substituiert sein kann; und
Het² für einen monocyclischen Heterocyclus ausgewählt aus Thiadiazolyl, Pyridinyl, Pyrimidinyl und Pyrazinyl steht.

2. Verbindungen nach Anspruch 1, wobei X für N steht, R¹ für Wasserstoff, C₁₋₆-Alkyl, Amino oder Di (C₁₋₆-alkyl)amino steht, R² für Wasserstoff steht, R³, R⁴ und R⁵ jeweils unabhängig voneinander aus Wasserstoff, Halogen, C₁₋₆-Alkyl, C₁₋₆-Alkyloxy, Trifluormethyl, Nitro und C₁₋₆-Alkyloxycarbonyl ausgewählt sind.

3. Verbindungen nach Anspruch 1 oder 2, wobei X für N steht, R¹ für Wasserstoff, C₁₋₄-Alkyl oder Di (C₁₋₄-alkyl)amino steht, R² für Wasserstoff steht, R³, R⁴ und R⁵ jeweils unabhängig voneinander aus Wasserstoff, Halogen, C₁₋₄-Alkyl, C₁₋₄-Alkyloxy oder Trifluormethyl ausgewählt sind und der zweiwertige Rest 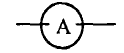 für Ar² , Ar²CH₂- oder Het² steht, wobei Ar² für Phenyl steht und Het² für Thiadiazolyl, Pyridinyl, Pyrimidinyl oder Pyrazinyl steht.

4. Verbindungen nach einem der Ansprüche 1 bis 3, wobei X für N steht, R¹ für Methyl steht, R² für Wasserstoff steht, R³ und R⁴ für Wasserstoff stehen und R⁵ für Trifluormethyl steht.

5. Verbindungen nach Anspruch 1, bei denen es sich um 1-[4-(3-Methyl-1,2,4-thiadiazol-5-yl)phenyl]-4-[3-(trifluormethyl)phenyl]piperazin, 1-[5-(3-Methyl-1,2,4-thiadiazol-5-yl)-2-pyridinyl]-4-[3-(trifluormethyl)phenyl]piperazin, stereoisomere Formen und pharmazeutisch unbedenkliche Säureadditionssalze davon handelt.

6. Zusammensetzung, enthaltend einen pharmazeutisch unbedenklichen Träger und, als Wirkstoff, eine therapeutisch wirksame Menge einer Verbindung nach einem der Ansprüche 1 bis 5.

7. Verfahren zur Herstellung einer pharmazeutischen Zusammensetzung nach Anspruch 6, bei dem man die pharmazeutisch unbedenklichen Träger und eine Verbindung nach einem der Ansprüche 1 bis 5 innig mischt.

8. Verbindungen nach einem der Ansprüche 1 bis 5 zur Verwendung als Medizin.

9. Verwendung einer Verbindung nach einem der Ansprüche 1 bis 5 zur Herstellung eines Medikaments zur Behandlung von angiogeneseabhängigen Erkrankungen.

10. Verfahren zur Herstellung einer Verbindung nach Anspruch 1, bei dem man
a) ein Zwischenprodukt der Formel (II) in einem reaktionsinerten Lösungsmittel und gegebenenfalls in Gegenwart einer geeigneten Base mit einem zwischenprodukt der Formel (III) umsetzt; 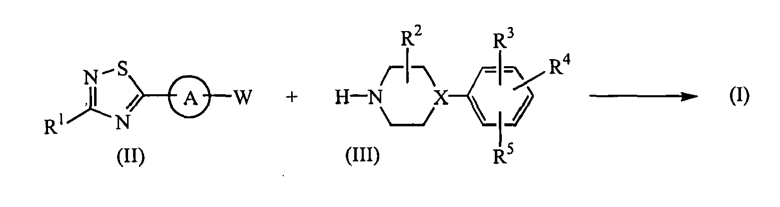
b) ein Zwischenprodukt der Formel (IV) in einem reaktionsinerten Lösungsmittel in Gegenwart einer geeigneten Base mit Hydroxylamino-O-sulfonsäure behandelt, was Verbindungen der Formel (I-a) liefert, die als Verbindungen der Formel (I) definiert sind, in denen R¹ für Methyl steht; 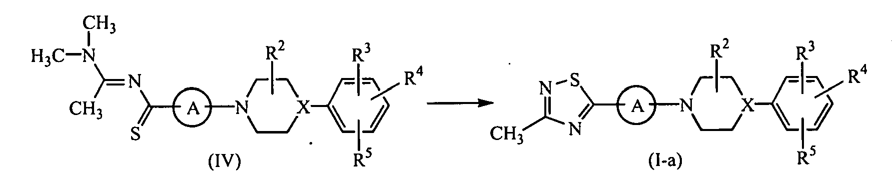
wobei in den obigen Reaktionsschemata die Reste X, R¹, R², R³, R⁴, R⁵ und 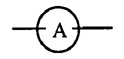 wie in Anspruch 1 definiert sind und W für eine geeignete Abgangsgruppe steht.

11. Verbindungen der Formel (IV) 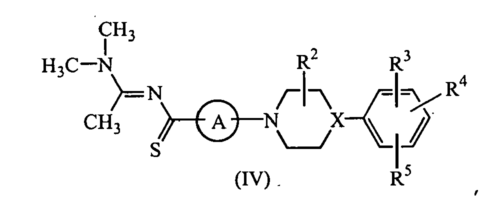 deren Säureadditionssalze, deren *N*-Oxidformen und deren stereochemisch isomere Formen, wobei X, R², R³, R⁴, R⁵ und der zweiwertige Rest 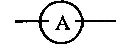 wie in Anspruch 1 definiert sind.

12. Verfahren zur Herstellung einer Verbindung der Formel (IV) nach Anspruch 10, bei dem man
a) ein zwischenprodukt der Formel (IX) in einem reaktionsinerten Lösungsmittel mit N,N-Dimethylacetamiddimethylacetal behandelt, was eine Verbindung der Formel (IV) liefert.

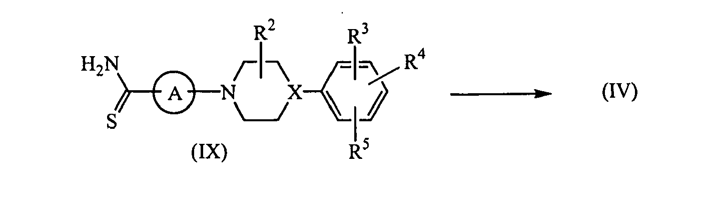

## Revendications

1. Composé de formule (I), 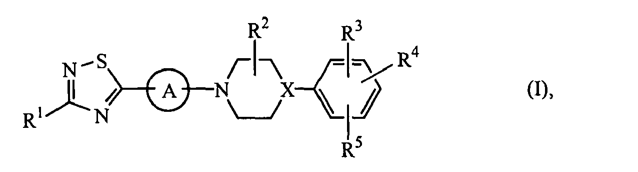 les formes N-oxyde, les sels d'addition pharmaceutiquement acceptables à un acide et les formes stéréochimiquement isomères de celui-ci, dans laquelle
X est un groupe CH ou atome d'azote ;
R¹ est un atome d'hydrogène, un groupe alkyle en C₁₋₆, un groupe alkyloxy en C₁₋₆, un groupe alkylthio en C₁₋₆, un groupe amino, un groupe mono- ou di (C₁₋₆alkyl) amino, un groupe Ar¹, un groupe Ar¹NH-, un groupe cycloalkyle en C₃₋₆, un groupe hydroxyméthyle ou un groupe benzyloxyméthyle ;
R² est un atome d'hydrogène ;
R³ R⁴ et R⁵ sont choisis chacun indépendamment parmi un atome d'hydrogène, un groupe halogéno, un groupe alkyle en C₁₋₆, un groupe alkyloxy en C₁₋₆, un groupe trifluorométhyle, un groupe nitro, un groupe amino, un groupe cyano, un groupe azido, un groupe C₁₋₆-alkyloxy-C₁₋₆-alkyle, un groupe alkylthio en C₁₋₆, un groupe C₁₋₆-alkyloxycarbonyle ou un groupe Het¹ ; 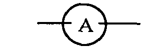 est un groupe Ar², un groupe Ar²CH₂- ou un groupe Het² ;
Ar¹ est un groupe phényle ; un groupe phényle substitué par 1, 2 ou 3 substituants choisis chacun indépendamment parmi un groupe halogéno, un groupe alkyle en C₁₋₆, un groupe alkyloxy en C₁₋₆, un groupe trihalogénométhyle, un groupe amino ou un groupe nitro ;
Ar² est un groupe phényle ;
Het¹ est un hétérocycle monocyclique choisi parmi un groupe oxazolyle, un groupe isoxazolyle, un groupe oxadiazolyle, un groupe thiazolyle, un groupe isothiazolyle, un groupe thiadiazolyle ou un groupe oxazolinyle ; et chaque hétérocycle monocyclique peut être éventuellement substitué sur un atome de carbone par un groupe alkyle en C₁₋₄ ; et
Het² est un hétérocycle monocyclique choisi parmi, un groupe thiadiazolyle, un groupe pyridinyle, un groupe pyrimidinyle ou un groupe pyrazinyle.

2. Composé selon la revendication 1, dans lequel X est un atome d'azote ; R¹ est un atome d'hydrogène, un groupe alkyle en C₁₋₆, un groupe amino ou un groupe di (C₁₋₆-alkyl) amino ; R² est un atome d'hydrogène ; R³, R⁴ et R⁵ sont choisis chacun indépendamment parmi un atome d'hydrogène, un groupe halogéno, un groupe alkyle en C₁₋₆, un groupe alkyloxy en C₁₋₆, un groupe trifluorométhyle, un groupe nitro ou un groupe C₁₋₆-alkyloxycarbonyle.

3. Composé selon l'une quelconque des revendications 1 ou 2, dans lequel X est un atome d'azote ; R¹ est un atome d'hydrogène, un groupe alkyle en C₁₋₄, ou groupe di(C₁₋₄-alkyl)amino; R² est un atome d'hydrogène ; R³, R⁴ et R⁵ sont choisis chacun indépendamment parmi un atome d'hydrogène, un groupe halogéno, un groupe alkyle en C₁₋₄, un groupe alkyloxy en C₁₋₄, un groupe trifluorométhyle ; et le radical bivalent 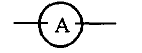 est un groupe Ar², un groupe Ar²CH₂- ou un groupe Het², où Ar² est un groupe phényle et Het² est un groupe thiadiazolyle, un groupe pyridinyle, un groupe pyrimidinyle ou un groupe pyrazinyle.

4. Composé selon l'une quelconque des revendications 1 à 3, dans lequel X est un atome d'azote, R¹ est un groupe méthyle, R² est un atome d'hydrogène, R³ et R⁴ sont un atome d'hydrogène et R⁵ est un groupe trifluorométhyle.

5. Composé selon la revendication 1, où le composé est la 1-[4-(3-méthyl-1,2,4-thiadiazol-5-yl)phényl]-4-[3-trifluorométhyl)phényl]-pipérazine ; ou
la 1-[5-(3-méthyl-1,2,4-thiadiazol-5-yl)-2-pyridinyl]-4-[3-(trifluorométhyl)phényl]-pipérazine ; une forme stéréoisomère ou un sel d'addition pharmaceutiquement acceptable à un acide de celui-ci.

6. Composition comprenant un support pharmaceutiquement acceptable, et en tant qu'ingrédient actif, une quantité thérapeutiquement efficace d'un composé selon l'une quelconque des revendications 1 à 5.

7. Procédé de préparation d'une composition pharmaceutique selon la revendication 6, dans lequel les supports pharmaceutiquement acceptables et un composé selon les revendications 1 à 5 sont mélangés intimement.

8. Composé selon l'une quelconque des revendications 1 à 5 destiné à être utilisé en tant que médicament.

9. Utilisation d'un composé selon l'une quelconque des revendications 1 à 5 pour la fabrication d'un médicament destiné au traitement de troubles dépendants de l'angiogenèse.

10. Procédé de préparation d'un composé selon la revendication 1, dans lequel
a) un intermédiaire de formule (II) est mis à réagir avec un intermédiaire de formule (III) dans un solvant inerte vis-à-vis de la réaction et éventuellement en présence d'une base appropriée ; 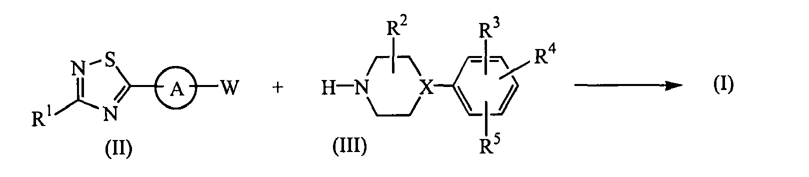
b) un intermédiaire de formule (IV) est traité avec de l'acide hydroxylamino-O-sulfonique dans un solvant inerte vis-à-vis de la réaction, en présence d'une base appropriée, en donnant ainsi lieu à des composés de formule (I-a), définis comme des composés de formule (I) dans laquelle R¹ est un groupe méthyle ; 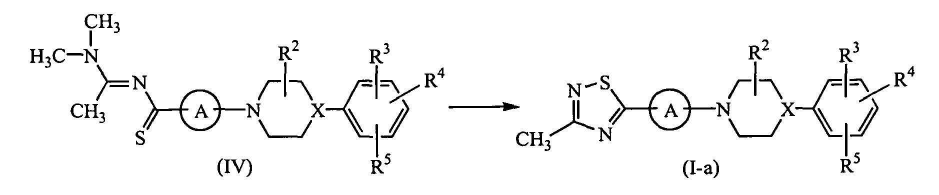
où dans les schémas réactionnels ci-dessus, les radicaux X, R¹, R², R³, R⁴, R⁵ et 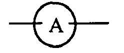 sont tels que définis dans la revendication 1, et W est un groupe partant approprié.

11. Composé de formule (IV), 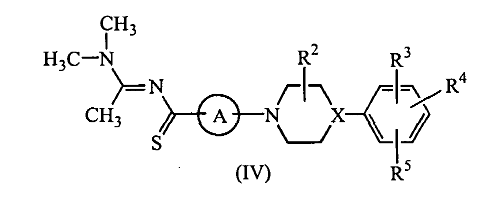 un sel d'addition à un acide, une forme N-oxyde ou une forme stéréochimiquement isomère de celui-ci, dans laquelle X, R², R³, R⁴, R⁵ et le radical bivalent 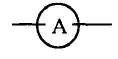 sont tels que définis dans la revendication 1.

12. Procédé de préparation d'un composé de formule (IV) selon la revendication 10, dans lequel
a) un intermédiaire de formule (IX) est traité avec du *N*,*N*-diméthylacétamide-diméthylacétal dans un solvant inerte vis-à-vis de la réaction, en donnant ainsi lieu à un composé de formule (IV) ;
